(19) Europäisches Patentamt
European Patent Office
Office européen des brevets

(11) **EP 4 455 665 A1**

(12) **EUROPEAN PATENT APPLICATION**
published in accordance with Art. 153(4) EPC

(43) Date of publication:
30.10.2024 Bulletin 2024/44

(21) Application number: 23820057.0

(22) Date of filing: 02.06.2023

(51) International Patent Classification (IPC):
*G01N 33/50* (2006.01)     *G16C 20/30* (2019.01)
*G16C 20/80* (2019.01)     *G16C 20/10* (2019.01)
*G16B 5/00* (2019.01)     *G16H 10/20* (2018.01)

(52) Cooperative Patent Classification (CPC):
**G01N 33/50; G16B 5/00; G16C 20/10; G16C 20/30;
G16C 20/80; G16H 10/20**

(86) International application number:
**PCT/KR2023/007615**

(87) International publication number:
**WO 2023/239121 (14.12.2023 Gazette 2023/50)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 09.06.2022  KR 20220070275
              29.08.2022  KR 20220108378
              13.04.2023  KR 20230048818

(71) Applicant: MBD Co., Ltd.
Suwon-si, Gyeonggi-do 16229 (KR)

(72) Inventors:
• KU, Bo Sung
  Yongin-si, Gyeonggi-do 16939 (KR)
• LEE, Sang-Hyun
  Suwon-si, Gyeonggi-do 16493 (KR)
• CHOI, Kwang Hak
  Suwon-si, Gyeonggi-do 16687 (KR)

(74) Representative: Wilding, Charlotte Katherine
**Venner Shipley LLP
5 Stirling House
The Surrey Research Park
Stirling Road
Guildford GU2 7RF (GB)**

(54) **METHOD AND SYSTEM FOR PREDICTING SENSITIVITY TO ANTI-CANCER AGENT**

(57)     The present invention relates to a method and system for predicting treatment responsiveness to an anticancer agent by using the CODRP index, which is an anticancer agent susceptibility index obtained by calculating Z-scores of each of the cell growth factor and drug response factor, integrating the same and converting the calculated values into Z-scores. Since the method for predicting susceptibility to an anticancer agent according to the present invention predicts susceptibility to an anticancer agent based on multiple factors of the drug response factor and cell growth factor, the prediction accuracy is improved compared to the conventional method for predicting susceptibility to an anticancer agent, and by predicting and providing appropriate treatment methods to individual cancer patients at an early stage, it has the advantage of reducing the pain of cancer patients during the treatment process by minimizing the trial and error of the medical personnel in selecting anticancer agents.

EP 4 455 665 A1

[Figure 2a]

**Description**

[Technical Field]

The present invention relates to a method and system for predicting susceptibility to an anticancer agent, and more specifically to a personalized method or system for predicting susceptibility to an anticancer agent, in which after performing cell culture of cancer organoids and treating with an anticancer agent, the Z-scores of each of a cell growth factor and a drug response factor, which are anticancer agent treatment responsiveness evaluation factors, are calculated, and the Z-scores of the values calculated by integrating the same are additionally calculated to use the derived anticancer agent susceptibility index.

[Background Art]

Cancer is generally treated by surgery, chemotherapy and radiation therapy, and among these, chemotherapy based on anticancer agents is mainly selected for cancer treatment, but the resistance of cancer cells to anticancer drugs has become the biggest obstacle to treating cancer with chemotherapy.

The resistance of cancer cells to anticancer agents can be explained in two ways, and the first way is intratumoral heterogeneity, which is a characteristic of cancer, and among cancer cells with various characteristics, cells with resistance to anticancer agents remain and show resistance (intrinsic resistance), and the second way is acquired resistance by causing genetic mutations in order to survive exposure to anticancer drugs, and these are direct factors in the failure of chemotherapy.

In particular, when chemotherapy is attempted for cancer patients, it is different for each patient which anticancer agent will exert a therapeutic effect or not, and if the anticancer agent does not exert a therapeutic effect, it is necessary to change the treatment method by replacing the same with another effective anticancer agent or trying a different treatment regimen before the cancer progresses further.

That is, the prediction of treatment responsiveness to an anticancer agent is essential for determining the treatment direction of cancer patients, and therefore, the situation is that the development of a method and system for predicting susceptibility to an anticancer agent that are capable of accurately predicting treatment responsiveness to an anticancer agent is continuously required.

In Korean Registered Patent No. 10-2343594, the method for predicting susceptibility to an anticancer agent based on the $IC_{50}$ value, which is defined as the concentration of a drug at which the cell activity is 50% based on cells showing 100% activity without drug treatment, has been newly proposed. In the above patent, the cell growth factor value reflecting the growth rate of cells can be reflected by considering the drug response factor value of an anticancer agent together with the cell growth rate as factors for predicting treatment responsiveness to an anticancer agent, and thus, it discloses that it is possible to overcome the limitations of the method for predicting susceptibility to an anticancer agent which uses only $IC_{50}$ value, which is a conventional drug response value.

However, when the drug response factor and cell growth factor of an anticancer agent are considered together, the accuracy of the method for predicting susceptibility to an anticancer agent is significantly improved compared to the conventional method for predicting susceptibility to an anticancer agent based on the $IC_{50}$ value, which has very poor prediction accuracy, but in the method for predicting susceptibility to an anticancer drug based on the anticancer drug sensitivity baseline, the criteria for distinguishing between anticancer drug sensitivity and resistance are not clear, and particularly, there was room for improvement in accurately determining susceptibility to an anticancer agent, such as the fact that the sample that was predicted to be resistant to an anticancer agent because it was located above the anticancer drug sensitivity baseline actually showed a 4% decrease in cancer tissue after cancer (sample #15).

Accordingly, the inventors of the present invention have made diligent efforts to develop a method for improving the accuracy of predicting susceptibility to an anticancer agent, and as a result, the present invention was completed by confirming that through the anticancer drug susceptibility index calculated by integrating the *Z-score* of a drug response factor and the *Z-score* of a cell growth factor, it is possible to predict susceptibility to an anticancer agent with significantly improved accuracy compared to the existing methods for predicting susceptibility to an anticancer agent.

[Disclosure]

[Technical Problem]

An object of the present invention is to provide a method for predicting susceptibility to an anticancer agent and a system for predicting susceptibility to an anticancer agent with improved accuracy, compared to the conventional methods for predicting susceptibility to an anticancer agent.

[Technical Solution]

The present invention provides a method for providing information for predicting susceptibility to an anticancer agent, including the following steps:

(a) culturing a biological sample isolated from a subject;
(b) calculating the Z-score ($Z_a$) of a drug response factor after treating the cultured biological sample with an anticancer agent, and calculating the Z-score ($Z_b$) of a cell growth factor by culturing the biological sample without treating an anticancer agent; and
(c) determining whether the anticancer agent response is positive or negative by integrating the calculated Z-score ($Z_a$) of the drug response factor and the Z-score ($Z_b$) of the cell growth factor.

In the method, the biological sample may be a cell sample isolated from a subject or a cell sample derived from a tissue isolated from a subject.

In the method, the cell sample may be three-dimensionally cultured in step (a).

In the method, step (c) may calculate the following CODRP index by integrating the calculated Z-score ($Z_a$) of the drug response factor and the Z-score ($Z_b$) of the cell growth factor:

$$\textbf{CODRP index} = Z - score\ of\ \sqrt{(Z_a + 4.5)^2 + (Z_b + 4.5)^2}\ .$$

In the method, the drug response factor may be $IC_{50}$ or an area of a drug response curve (AUC)

In the method, when the CODRP Index is -0.5 or less, the anticancer agent response may be determined as positive, and when the CODRP Index is 0 or more, the anticancer agent response may be determined negative, and when the CODRP Index is greater than -0.5 and less than 0, it may be determined as deferred.

In addition, the present invention provides a system for predicting susceptibility to an anticancer agent, including:

a receiving unit which is configured to receive cell experiment-based biological test data for a biological sample isolated from a subject;
a processor which is connected to the receiving unit; and
an output unit for outputting a result processed by the processor,
wherein the processor calculates and integrates the Z-score ($Z_a$) of a drug response factor and the Z-score ($Z_b$) of a cell growth factor among the biological test data to provide prediction results of susceptibility to an anticancer agent for the subject.

In the system, the processor may calculate and integrate the Z-score ($Z_a$) of a drug response factor and the Z-score ($Z_b$) of a cell growth factor to calculate the following CODRP index:

$$\textbf{CODRP index} = Z - score\ of\ \sqrt{(Z_a + 4.5)^2 + (Z_b + 4.5)^2}\ .$$

In the system, the drug response factor may be $IC_{50}$ or an area of a drug response curve (AUC)

In the system, when the CODRP Index is -0.5 or less, the processor may determine the anticancer agent response as positive, and when the CODRP Index is 0 or more, the processor may determine the anticancer agent response as negative, and when the CODRP Index is greater than -0.5 and less than 0, the processor may determine as deferred.

In the system, the cell experiment-based biological test data may include test data of at least one of the name of an anticancer agent, the concentration of an anticancer agent, a cell growth rate and the degree of apoptosis.

In addition, the present invention provides a computer-readable recording medium which records a program for implementing the system for predicting susceptibility to an anticancer agent.

[Advantageous Effects]

Since the method for predicting susceptibility to an anticancer agent according to the present invention predicts susceptibility to an anticancer agent based on multiple factors of the drug response factor and cell growth factor, the prediction accuracy is improved compared to the conventional method for predicting susceptibility to an anticancer agent, and by predicting and providing appropriate treatment methods to individual cancer patients at an early stage, it has the advantage of reducing the pain of cancer patients during the treatment process by minimizing the trial and error of the medical personnel in selecting anticancer agents.

[Description of Drawings]

FIG. 1a shows a DRC graph for calculating $IC_{50}$.

FIG. 1b shows a DRC graph for calculating AUC.

FIG. 2a shows the process of calculating the CODRP index according to the present invention. The X-axis represents the Z-score ($Z_a$) of a drug response factor, and the Y-axis represents the Z-score ($Z_b$) value of a cell growth factor. The distance (d) was calculated according to the calculated $Z_a$ and $Z_b$ values of the sample by using (-4.5, -4.5) as a reference point, and the Z-score of this distance was named as the CODRP Index, which is an anticancer agent susceptibility index.

FIG. 2b is the results of analyzing the recurrence prediction in ovarian cancer patients and the p-value of the disease free survival (DFS) of the responder group (sensitive group) and the non-responder group (resistant group) in order to derive the cut-off value of the CODRP Index.

FIGS. 3a to 3c are the results of comparing the sensitivity and specificity of the method for predicting susceptibility to an anticancer agent according to the present invention with the conventional method for predicting susceptibility to an anticancer agent in breast cancer, lung cancer and colorectal cancer patient samples, respectively, and FIG. 3d is the results of integrating the same.

FIGS. 4a to 4c are the results of comparing the correlation between $IC_{50}$ and AUC and drug response in the method for predicting susceptibility to an anticancer agent to Paclitaxel, Carboplatin and Paclitaxel+Carboplatin by using ovarian cancer patient samples.

FIG. 5 is the results of comparing prediction rates for relapsed patients in Comparative Example 2 and Example 2 in the method for predicting susceptibility to an anticancer agent for Carboplatin and Paclitaxel+Carboplatin by using ovarian cancer patient samples. Red circles indicate recurrent patients.

FIG. 6 is the results of comparing p-values of the disease free survival (DFS) of Comparative Example 2 and Example 2 in the method for predicting susceptibility to an anticancer for Carboplatin and Paclitaxel+Carboplatin by using ovarian cancer patient samples.

FIG. 7 is the results of comparing p-values of the probability of survival in Example 1 and Example 2 in the method for predicting susceptibility to an anticancer agent for Paclitaxel+Carboplatin by using ovarian cancer patient samples.

[Modes of the Invention]

Unless defined otherwise, all technical and scientific terms used herein have the same meanings as commonly understood by one of ordinary skill in the art to which the present invention pertains. In general, the nomenclature used herein and the experimental methods described below are those well-known and commonly used in the art.

In the present invention, attempts have been made to improve the accuracy of conventional methods for predicting susceptibility to an anticancer agent, and a novel method and system that are capable of accurately predicting susceptibility to an anticancer agent by calculating the Z-score of each of a drug response factor and a cell growth factor and integrating the same were developed.

Therefore, in one aspect, the present invention relates to a method for predicting susceptibility to an anticancer agent, including the following steps, for example, a method for providing information for predicting susceptibility to an anticancer agent:

(a) culturing a biological sample isolated from a subject;

(b) calculating the Z-score ($Z_a$) of a drug response factor after treating the cultured biological sample with an anticancer agent, and calculating the Z-score ($Z_b$) of a cell growth factor by culturing the biological sample without treating an anticancer agent; and

(c) determining whether the anticancer agent response is positive or negative by integrating the calculated Z-score ($Z_a$) of the drug response factor and the Z-score ($Z_b$) of the cell growth factor.

In the above method, the biological sample may be a cell sample which is isolated from a subject or a cell sample which is derived from a tissue isolated from a subject, but the present invention is not limited thereto.

The method may be characterized as predicting *ex vivo,* for example, *in vitro.*

In the method, the cell sample in step (a) may be characterized in that it is three-dimensionally cultured, but the present invention is not limited thereto.

In this case, the cell sample may be cultured on a culture plate including a plurality of pillars that protrude in a columnar shape to have a certain height from the flat floor, as a portion in which an accommodation space in which a cell culture solution can be accommodated is formed and cells, which are culture subjects, are placed, and a trench groove which is formed concavely opposite to the pillars that are formed convexly with respect to the flat floor such that the culture solution placed in the accommodation space can be gathered on one side without being scattered on the flat floor.

Next, biological test data may be obtained after a biological test for predicting susceptibility to an anticancer agent is performed on the cells cultured on the culture plate.

In this case, with regard cell culture, Korean Patent Application Laid-Open No. 10-2020-0055230 is attached as a reference. Meanwhile, the culture plate and culture conditions for culturing cells are not limited to the above-described aspects.

In the above method, step (c) may be characterized in that the following CODRP index is calculated by integrating the calculated Z-score ($Z_a$) of a drug response factor and the Z-score ($Z_b$) of a cell growth factor, but the present invention is not limited thereto:

$$\textbf{CODRP index} = Z - score\ of\ \sqrt{(Z_a + 4.5)^2 + (Z_b + 4.5)^2}$$

In the method, the drug response factor may be $IC_{50}$ or an area of drug response curve (AUC), but the present invention is not limited thereto.

Particularly, in the present invention, when the drug response factor is an area (AUC) of the drug response curve, since it reflects the drug response according to the actual results at all concentrations, accurate drug response evaluation is made, and since $IC_{50}$ can be calculated only when the cell viability is 50% in the experimental results, the AUC can reflect the responsiveness of cancer cells to drugs more efficiently. If a perfect linear interval does not appear depending on the drug concentration, accurate drug response evaluation may not be performed because the $IC_{50}$ has to be obtained by relying on an approximate trend line. On the other hand, the AUC reflects the drug's response according to the actual experimental results at all concentrations, and accurate drug response evaluation is performed.

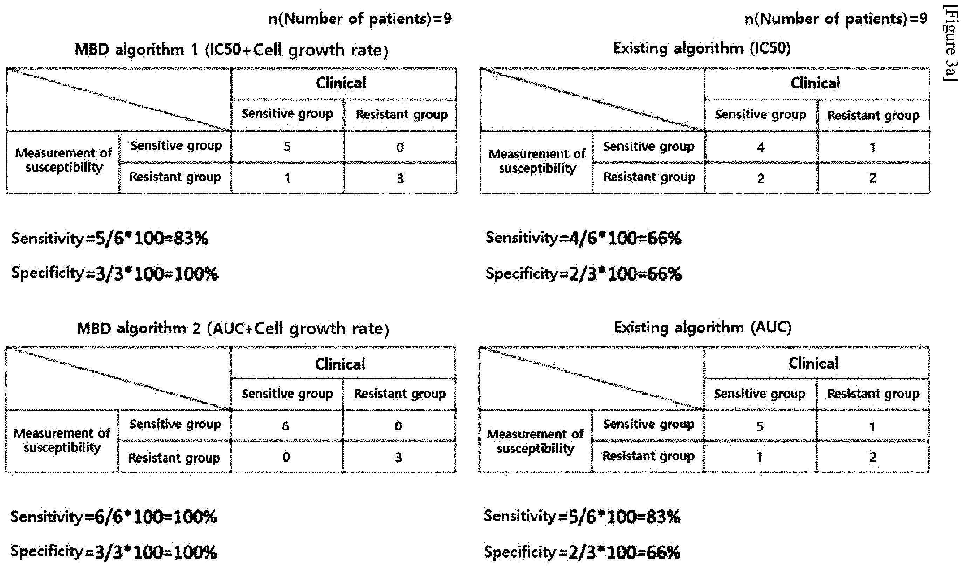

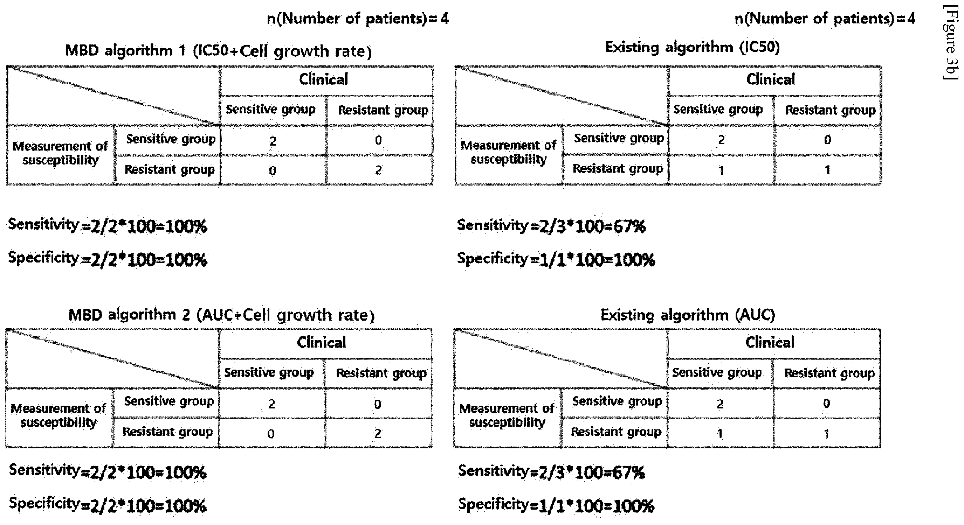

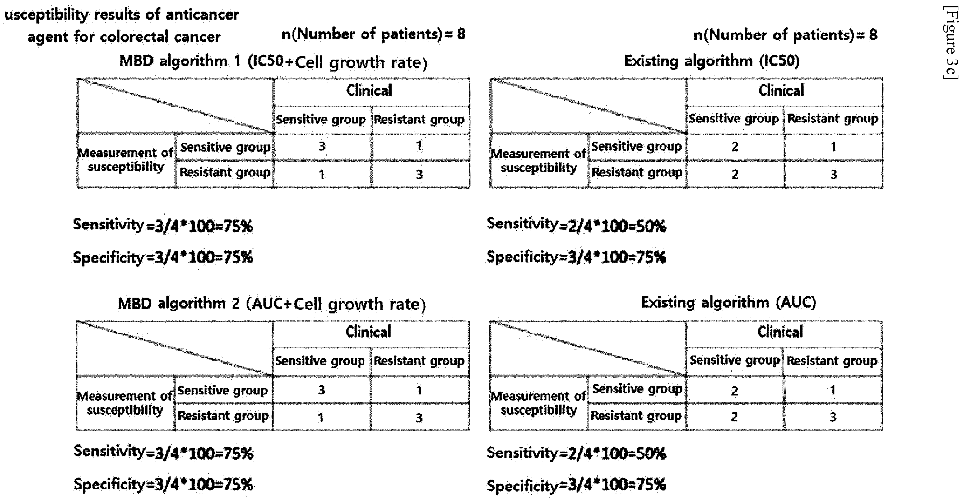

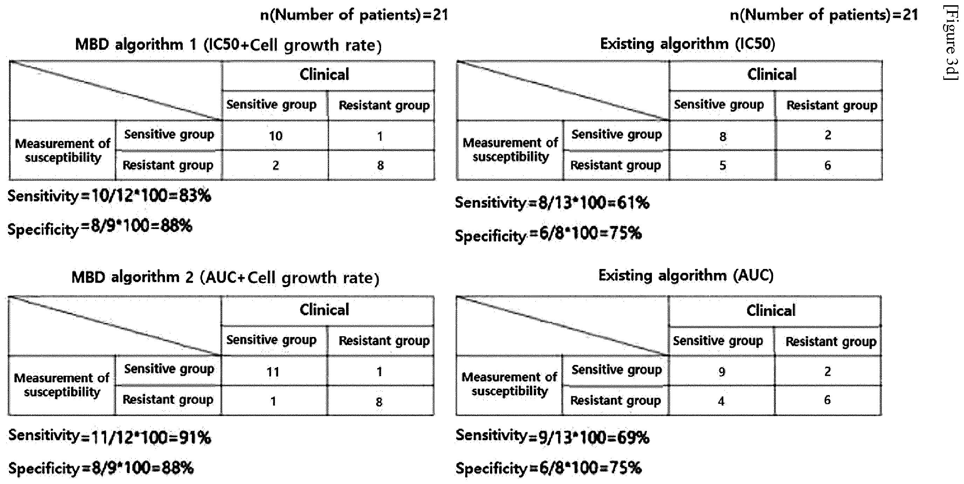

Therefore, in the present invention, the drug response factor may be $IC_{50}$ or an area of the drug response curve (AUC), but when the area of the drug response curve (AUC) is applied rather than the $IC_{50}$, it has been confirmed that more accurate prediction for susceptibility to an anticancer agent is possible. (FIGS. 3A to 3D).

In the above method, when the CODRP Index is -0.5 or less, the anticancer agent response is determined as positive, and when the CODRP Index is 0 or more, the anticancer agent response is determined as negative, and when the CODRP Index is greater than -0.5 and less than 0, it is determined as deferred.

In another aspect, the present invention provides a system for predicting susceptibility to an anticancer agent, including:

> a receiving unit which is configured to receive cell experiment-based biological test data for a biological sample isolated from a subject;
> a processor which is connected to the receiving unit; and
> an output unit for outputting a result processed by the processor,
> wherein the processor calculates and integrates the Z-score ($Z_a$) of a drug response factor and the Z-score ($Z_b$) of a cell growth factor among the biological test data to provide prediction results of susceptibility to an anticancer agent for the subject.

In the system, the processor may calculate and integrate the Z-score ($Z_a$) of a drug response factor and the Z-score ($Z_b$) of a cell growth factor to calculate the following CODRP index:

$$\textbf{CODRP index} = Z - score\ of\ \sqrt{(Z_a + 4.5)^2 + (Z_b + 4.5)^2}$$

In the system, the drug response factor may be $IC_{50}$ or an area of drug response curve (AUC), but the present invention is not limited thereto.

In the system, when the CODRP Index is -0.5 or less, the processor determines the anticancer agent response as positive, and when the CODRP Index is 0 or more, the processor determines the anticancer agent response as negative, and when the CODRP Index is greater than -0.5 and less than 0, the processor determines as deferred.

In the system, the cell experiment-based biological test data may include test data of at least one of an anticancer agent name, an anticancer agent concentration, a cell growth rate and the degree of apoptosis.

Meanwhile, the method and system for predicting susceptibility to an anticancer agent according to the present invention have been improved to enhance the prediction accuracy of Korean Registered Patent No. 10-2343594, and within the scope of not contradicting the contents of the present invention, the entire text of the above patent may be referenced as some embodiments for practicing the present invention.

In the present invention, the system for predicting susceptibility to an anticancer agent may be a system which is configured to provide information related to susceptibility to an anticancer agent based on biological test data for a subject.

In one aspect, the system for predicting susceptibility to an anticancer agent may be integrated with a database providing server that provides biological test data and/or clinical result standard data, and/or a user system that receives information on susceptibility to an anticancer agent.

In this case, the system for predicting susceptibility to an anticancer agent may include a general-purpose computer, a laptop and/or a data server for performing various calculations to predict the degree of susceptibility to an anticancer agent, based on the user's biological test data and/or clinical result standard data provided from the database providing server.

More specifically, the system for predicting susceptibility to an anticancer agent of the present invention may receive biological test data from a database providing server and provide prediction results of susceptibility to an anticancer agent derived from the received biological test data. For example, the system for predicting susceptibility to an anticancer agent according to the present invention predicts treatment responsiveness when an anticancer agent A is used for the subject, by providing the subject with an anticancer agent susceptibility index for the anticancer agent A, that is, the CODRP index.

In this case, the database may collect and store drug response factors and cell growth factor data from a plurality of patients having the same carcinoma, and these can be used to provide average and standard deviation information for calculating the CODRP index in the system for predicting susceptibility to an anticancer drug according to the present invention.

Meanwhile, the user system is an electronic system that requests the provision of prediction results of susceptibility to an anticancer agent for a subject and provides a user interface for displaying the prediction results, and it may include at least one of a smart phone, a tablet PC (Personal Computer), a laptop and/or a PC.

That is, the user system is a means that is implemented such that the output unit in the system for predicting susceptibility to an anticancer agent can easily check the prediction results of susceptibility to an anticancer agent for the subject by a medical staff treating the subject, and the user system may be a system for accessing a web server for providing web pages or a mobile web server providing mobile web sites, but the present invention is not limited thereto.

In this case, the results provided from the system for predicting susceptibility to an anticancer agent may be provided as a web page through a web browser installed in a user system, or may be provided in the form of an application or program. In various exemplary embodiments, this data may be provided in the form of being included in the platform in a client-server environment.

In one aspect, the user system may receive the prediction results of susceptibility to an anticancer agent for a subject from the system for predicting susceptibility to an anticancer agent, and display the received results through a display unit. Herein, the prediction results may include the degree of susceptibility to the anticancer agent A (e.g., high, medium or low, treatment response positive or treatment response negative) that is output by the system for predicting susceptibility to an anticancer agent, and may further include the CODRP index and the like for the anticancer agent A to determine the same.

Meanwhile, another embodiment of the system for predicting susceptibility to an anticancer agent of the present invention may further include a storage unit and a communication unit.

First of all, the storage unit may store various data generated in the process of predicting the degree of susceptibility to an anticancer agent for a subject. For example, the storage unit may be configured to store biological test data or susceptibility characteristics extracted from biological test data, and further susceptibility prediction results. In various embodiments, the storage unit may include at least one type of storage medium among a flash memory type, a hard disk type, a multimedia card micro type, a card type memory *(e.g.,* SD or XD memory, *etc.),* RAM, SRAM, ROM, EEPROM, PROM, magnetic memory, magnetic disk and optical disk.

The communication unit connects the system for predicting susceptibility to an anticancer agent and an external system to enable communication. The communication unit may transmit/receive various data by being connected to a user system and further a database providing server by using wired/wireless communication. Specifically, the communication unit may receive biological test data and clinical result reference data of a subject from the database providing server. Furthermore, the communication unit may transmit the prediction results to the user system.

The processor is operatively connected to the receiving unit, the storage unit, the output unit and/or the communication unit, and it is possible to analyze biological test data for a subject to extract associated susceptibility characteristics, and based on the same, various instructions for determining the degree of susceptibility to an anticancer agent may be performed.

Specifically, the processor may be configured to classify susceptibility characteristics based on the biological test data received through the receiving unit or the communication unit, and predict the degree of susceptibility to an anticancer agent. For example, the susceptibility characteristics may be a fitting line generated by curve fitting biological test data including a cell growth factor and a drug response factor through regression analysis. In this case, the cell growth factor is a concept that encompasses all Z-scores (statistical indicators) of factors that can indicate cell proliferation, such as cell growth rate, and the drug response factor is a concept that encompasses all Z-scores (statistical indicators) of factors that can indicate anticancer drug efficacy, such as $IC_{50}$, $\%IC_{50}$, AUC (area of drug response curve), %AUC and cell activity.

Meanwhile, the system for predicting susceptibility to an anticancer agent is not limited to those designed in hardware. For example, the processor of the system for predicting susceptibility to an anticancer agent may be implemented as software. Therefore, the prediction results of susceptibility to an anticancer agent may be displayed through a display unit of the user system to be described below.

In the present invention, an embodiment of the user system includes a communication unit, a display unit, a storage unit and a processor.

The communication unit may be configured to allow the user system to communicate with an external system. The communication unit may be connected to the system for predicting susceptibility to an anticancer agent by using wired/wireless communication to transmit various data related to the susceptibility to an anticancer agent. Specifically, the communication unit may receive prediction results of susceptibility to an anticancer agent for a subject from the system for predicting susceptibility to an anticancer agent, such as an efficacy evaluation chart of an anticancer agent showing the degree of susceptibility (high, medium or low) of the subject to the anticancer agent, positive treatment response, and further the degree of sensitivity to an anticancer agent as a chart.

In various embodiments, the display unit may include a touch screen, and for example, it may receive a touch using an electronic pen or a part of the user's body, a gesture, proximity, drag, swipe, a hovering input or the like.

The storage unit may store various data that is used to provide a user interface for displaying result data. In various embodiments, the storage unit may include at least one type of storage medium among a flash memory type, a hard disk type, a multimedia card micro type, a card type memory (e.g., SD or XD memory, *etc.*), RAM (Random Access Memory), SRAM (Static Random Access Memory), ROM (Read Only Memory), EEPROM (Electrically Erasable Programmable Read-Only Memory), PROM (Programmable Read-Only Memory), magnetic memory, magnetic disk and optical disk.

The processor is operatively connected to the communication unit, the display unit and the storage unit, and it may perform various commands to provide a user interface for displaying result data.

Hereinafter, the method for predicting susceptibility to an anticancer agent according to an embodiment of the present invention will be described in detail.

The procedure for predicting susceptibility to an anticancer agent according to an embodiment of the present invention is as follows. First of all, biological test data for a subject is received. Next, based on the biological test data, the subject's susceptibility to an anticancer agent is predicted. Finally, the prediction results of susceptibility to an anticancer agent are provided.

According to a feature of the present invention, in the step of receiving the biological test data, the biological data, for example, biological test results and test condition data for predicting the efficacy of an anticancer drug for cancer cells isolated from a subject who has developed ovarian cancer, breast cancer or colorectal cancer may be received.

Preferably, in the step of receiving the biological test data, the biological test data for cancer cells that are cultured on a culture plate including a plurality of pillars that protrude in a columnar shape to have a certain height from the flat floor, as a portion in which an accommodation space in which a cell culture solution can be accommodated is formed and cells, which are culture subjects, are placed, and a trench groove which is formed concavely opposite to the pillars that are formed convexly with respect to the flat floor such that the culture solution placed in the accommodation space can be gathered on one side without being scattered on the flat floor, but the present invention is not limited thereto.

Meanwhile, according to another feature of the present invention, prior to the step of receiving the biological test data, a step of authenticating a user who wants to be provided with the predictive result of susceptibility to an anticancer agent for a subject may be further performed.

That is, in the step of receiving the biological test data, after the authenticated user logs in, the biological test data for the subject may be received.

Next, in the step of predicting susceptibility to an anticancer agent, the susceptibility to an anticancer agent for a subject may be determined as treatment response positive or treatment response negative by the system for predicting susceptibility to an anticancer agent which is configured to output the degree of susceptibility to an anticancer agent by having the drug response factor and the cell growth factor as inputs.

Meanwhile, in the system for predicting susceptibility to an anticancer agent, the susceptibility to an anticancer agent may be determined by the CODRP Index which is calculated by calculating the Z-scores of each of the cell growth factor and the drug response factor and integrating the same.

In this case, in the processor of the system for predicting susceptibility to an anticancer agent, when the CODRP Index is -0.5 or less, it may determine the anticancer agent response as positive, and when the CODRP Index is 0 or more, it may determine the anticancer agent response as negative, and when the CODRP Index is greater than -0.5 and less than 0, it may determine as deferred.

In another aspect of the present invention, the receiving unit of the system for predicting susceptibility to an anticancer agent may receive the biological test data and the clinical result standard data, and the receiving unit of the system for predicting susceptibility to an anticancer agent may provide the prediction results of susceptibility to an anticancer agent for a subject based on the biological test data and the clinical result standard data.

In the present invention, for example, after the user is authenticated through log-in, the received biological test data is input into an input module of the system for predicting susceptibility to an anticancer agent and then received by the receiving unit, and then, the susceptibility to an anticancer agent is analyzed from the biological test data in the processor by an analysis module, and the analyzed result may be output by an output module in the output unit. In this case, the output module may predict the susceptibility to an anticancer agent for the subject by using the clinical standard result data together with the susceptibility characteristics extracted from the biological test data. In this case, the susceptibility to an anticancer agent may be expressed as a degree of treatment response, and an analysis result may be provided by determining the same as high, medium or low. More specifically, the analysis module calculates the CODRP index from the biological test data, and the output module determines the degree of treatment response as 'high' when the CODRP index is less than -0.5, determines the degree of treatment response as 'middle' when the CODRP index is more than -0.5 and is less than 0, and determines the degree of treatment response as 'low' when the CODRP index is 0 or more.

However, the method for predicting susceptibility to an anticancer agent is not limited thereto, and the output module may output a treatment response positive or a treatment response negative, or may stochastically output a treatment response. Furthermore, the output module may further determine an efficacy prediction chart of the anticancer agent representing the degree of susceptibility to the anticancer agent as a chart.

Meanwhile, according to another feature of the present invention, user logout may be performed after the step of providing prediction results of susceptibility to an anticancer agent.

Meanwhile, in another aspect, the present invention may be provided as a computer-readable recording medium for recording a program for implementing the system for predicting susceptibility to an anticancer agent.

For the clarity of interpretation of the present specification, terms used in the present specification will be defined below.

As used herein, the term "subject" may refer to any subject whose susceptibility to an anticancer agent is to be predicted. For example, a subject may be an individual who has developed ovarian cancer, breast cancer, squamous cell carcinoma, uterine cancer, cervical cancer, prostate cancer, head and neck cancer, pancreatic cancer, brain tumor, liver cancer, skin cancer, esophageal cancer, testicular cancer, kidney cancer, colorectal cancer, rectal cancer, lung cancer, stomach cancer, kidney cancer, bladder cancer, bile duct cancer and gallbladder cancer. Preferably, the subject may be an individual whose susceptibility to an anticancer agent of doxorubicin is to be predicted, such as an individual who has developed ovarian cancer or breast cancer, an individual whose susceptibility to an anticancer agent of Osmertinib and/or Afatinib is to be predicted, such as an individual who ha developed lung cancer, an individual whose susceptibility to an anticancer agent of 5-FU and/or Oxaliplatin is to be predicted, such as an individual who has developed colorectal cancer, and an individual whose susceptibility to an anticancer agent of Paclitaxel and/or Carboplatin is to be predicted, such as an individual who has developed ovarian cancer, but the present invention is not limited thereto. Furthermore, the subject disclosed herein may be a mammal, but the present invention is not limited thereto.

As used herein, the term "anticancer agent" is a chemotherapeutic agent for inhibiting the proliferation of cancer cells, and it may include chemo-cancer agents and targeted anticancer agents. For example, the anticancer agent may be Paclitaxel including Doxorubicin, Taxotere, Adriamycin, Endostatin, Angiostatin, Mitomycin, Bleomycin, Cisplatin, Carbopletin, Daunorubicin, Idarubicin, 5-Florouracil, Methotrexate, Actinomycin-D or a mixture thereof. Preferably, the anticancer agent within the present specification may be Doxorubicin, but the present invention is not limited thereto.

As used herein, the term "susceptibility to an anticancer agent" may refer to sensitivity or responsiveness to an anticancer agent, and it may be a measure for predicting whether a therapeutic response is exhibited according to treatment with a target anticancer agent. In this case, within the present specification, the susceptibility to an anticancer agent sensitivity may be used interchangeably with treatment responsiveness of anticancer agents, drug responsiveness and the like. As such, according to prediction of susceptibility to an anticancer agent, the subject may be determined as a treatment-positive individual (anticancer drug-responsive individual) or a treatment-negative individual (anticancer drug-resistant individual) for a specific anticancer agent. More specifically, a subject having a relatively high susceptibility to an anticancer agent for a target anticancer agent may be determined to have a positive treatment response, and a subject having a relatively low susceptibility to an anticancer agent for a target anticancer agent may be determined to have a negative treatment response.

In this case, the susceptibility to an anticancer agent may be associated with the growth rate of cancer cells together with $IC_{50}$ or AUC, which are drug response factors for cancer cells that are isolated from the subject. More specifically, the degree of drug response (degree of drug efficacy) of cancer cells may be inversely proportional to the growth rate of cancer cells. That is, when the cell experiment-based drug response factor and the growth rate of cancer cells, which are quantitative indicators of efficacy evaluation, are reflected in predicting the susceptibility to an anticancer agent, the accuracy and sensitivity of prediction of the susceptibility to an anticancer agent may be higher than when only a single factor is considered. In particular, the prediction results of susceptibility to an anticancer agent based on multiple factors such as the drug response factor and the cell growth factor may be highly accurate enough to be matched with the clinical results of an individual.

As used herein, the term "drug response factor" is a measure for quantitatively measuring the response of a cell-based drug and includes the activity of a cell according to a drug. In the present invention, the drug may be an anticancer agent.

For example, the activity of cells may be measured by staining live cells to measure the total intensity/size, selected intensity/size and the like of a fluorescent substance, or by staining dead cells to obtain apoptosis and conversely measuring the activity of cells.

In addition, it may include measuring the cell activity by measuring the transformation of cells or a solution containing the cells by using a chemical element such as MTT or ATP.

As used herein, the term "cell growth factor" includes quantitative indicators that measure the growth of cells over time. For example, the cell growth factor may be obtained by measuring the activity of cells over time.

In this case, the growth factor may be a cell activity increase rate, a cell growth rate, a cell size increase rate or a colony generation rate. As used herein, the term "cell growth rate" may refer to changes in cell volume, size and number for a given period of time.

In this case, the cell growth rate may be calculated only for specific cells. The growth factor of cells may be calculated by confining cell division or growth-active cell groups among various cells.

As used herein, the term "biological test data" may refer to cell experiment-based test result data or test conditions for a biological sample that is isolated from a subject. In this case, the biological sample may be at least one of tissue, cell, pleural fluid, ascites, whole blood, serum, plasma, saliva, cerebrospinal fluid and urine.

Preferably, the biological test data within the present specification may be test data and/or test condition data on cancer cells (or cancer tissues) that are isolated from a subject. For example, the biological test data may be raw data associated with at least one information of the name of an anticancer agent, the concentration of an anticancer agent, the cell growth rate and the degree of apoptosis, and furthermore, the anticancer agent treatment condition data. However, the biological test data is not limited to the foregoing description. As used herein, the term "about" may be interpreted as meaning approximately, about, roughly or to a certain extent. When the term "about" is used with a numerical range, it shall be interpreted as modifying that range by extending the boundaries above and below the specified numerical value. In general, the term "about" is used herein to modify a numerical value above and below the specified value by a variance of 10%.

The terms "individual," "patient" and "subject" are used interchangeably and include mammals such as mice, rats, other rodents, rabbits, dogs, cats, pigs, cattle, and sheep, horses or any animal including primates including humans.

Example

Hereinafter, the present invention will be described in more detail through examples. These examples are only for illustrating the present invention, and it will be apparent to those skilled in the art that the scope of the present invention is not to be construed as being limited by these examples.

**Example 1. Development of method for predicting susceptibility to anticancer agent**

Experiments to predict the susceptibility to an anticancer agent were conducted in the following steps: pretreatment → 3D culture and anticancer agent treatment → cell death (activity) calculation → cell growth rate calculation → result analysis.

1-1. Pretreatment

After removing cancer tissue through surgery or biopsy, it was transferred to the laboratory while being refrigerated in Transfer Media (Table 1). The outside of the transported specimen was disinfected with 70% ethanol, and the transport time and specimen conditions were visually checked. After cutting the sample tissue finely with a blade in the clean bench, it was placed in a Falcon Tube by using Dissociation Buffer (Table 2) including the composition according to the manufacturer's instructions, and then reacted in a $CO_2$ incubator at 37°C for 1 hour.

[Table 1]

| Transfer media component | Vandor | Final conc. |
|---|---|---|
| adv. DMEM/F12 | Gibco | 1X |
| P/S | invitrogen | 100u/ml, 100ug/ml |
| Glutamax (100X) | invitrogen | 1X |
| HEPES | Invitrogen | 10mM |
| Primocin (50mg/ml) | Invivogen | 50ug/ml |
| Human serum | Thermo Fiseher | 2% |
| Rock inhibitor Y-27632 (10mM) | abmole | 5uM |

[Table 2]

| Dissociation Buffer (Breast cancer/Lung cancer) | Vandor |
|---|---|
| adv. DMEM/F12 | Gibco |
| penicillin streptomycin(antibiotics) | invitrogen |
| Dnase I * | Merck |
| Collagenase/Hyaluronidase(3000U/ml, 1000U/ml) | STEMCELL |
| | |
| Dissociation Buffer (Colorectal cancer/Ovarian cancer) | Vandor |
| HBSS | Gibco |
| penicillin streptomycin(antibiotics) | Invitrogen |
| DNase I | Merck |
| Dispase 2 | Gibco |
| Collagenase I | Gibco |
| Rock inhibitor, Y-27632 | abmole |

Cells were collected by using a centrifuge, the supernatant was carefully removed, and 10 mL of PBS was added and washed again by centrifugation. After releasing the cells by adding 1 mL of the culture solution (Table 3), the cells were counted by using a cytometer.

[Table 3]

| component (final conc.) | Breast cancer | Lung cancer | Colorectal cancer (PDXO) | Ovarian cancer |
|---|---|---|---|---|
| adv. DMEM/F12 | 1X | 1X | 1X | 1X |
| P/S | 100u/ml, 100ug/ml | 100u/ml, 100ug/ml | 100u/ml, 100ug/ml | 100u/ml, 100ug/ml |
| Glutamax (100X) | 1X | 1X | 1X | 1X |
| HEPES | 10mM | 10mM | 10mM | 10mM |
| Primocin | 50ug/ml | 50ug/ml | 100ug/ml | - |
| R-spondin 1 | - | - | 500mg/ml -->1 ug/ml | 1 ug/ml |

(continued)

| component (final conc.) | Breast cancer | Lung cancer | Colorectal cancer (PDXO) | Ovarian cancer |
|---|---|---|---|---|
| R-spondin 3 | 250ng/ml | 250ng/ml | - | - |
| Noggin | 100ng/ml | 100ng/ml | 100ng/ml | 100ng/ml |
| Y-27632 | 5uM | 5uM | 10uM | 10 uM |
| A 83-01 | 500nM | 500nM | 500nM | 500nM |
| EGF | 5 ng/ml | 5ng/ml | 50ng/ml | - |
| B-27 supplement(50X) | 1X | 1X | 1X | 1X |
| N-acetyl-L-Cysteine | 1.25mM | 1.25mM | 1.25mM | 1.25mM |
| FGF7 | 5 ng/ml | 5ng/ml | - | - |
| FGF10 | 20ng/ml | 20ng/ml | - | - |
| Nicotiamide | 5mM | 5mM | 10mM | - |
| Neuregulin 1 | 5nM | 5nM | - | - |
| Leu15-Gas-trin I | - | - | 10nM | 10 nM |
| Recombinant Human IGF-1 (Insulin) | - | - | - | 100 ng/ml |
| Recombinant Human FGF-2 | - | - | - | 50 ng/ml |
| Afamin/Wnt3a CM | - | - | - | 10% |
| b-estradiol | 160pM | - | - | - |
| SB202190 | - | - | 5uM | - |
| PGE2 | - | - | 1uM | - |

1-2. 3D culture and treatment with anticancer agents

The concentration was calculated as 2X according to the number of cells, and the same amount of Matrigel (Matrigel-GFR (Corning), BME (Cultrex)) was prepared and mixed at 1:1 to make the final concentration 1X. A 384-well plate (SPL) was prepared, and the culture solution in Table 2 was dispensed. The cell and Matrigel mixture was dispensed into the plate well, and the discharged pillar plate (refer to Korean Patent Application Laid-Open No. 10-2020-0055230) was combined with the well plate containing the medium. After stabilization by culturing in a $CO_2$ incubator at 37°C for 3 days, the pillar plate was moved to a well plate in which the anticancer agent or PBS or DMSO (control group)-treated culture solution was dispensed such that the cells were exposed to the anticancer agent and cultured for 5 to 10 days in a $CO_2$ incubator at 37°C.

1-3. Calculation of the degree of apoptosis (activity)

1-3-1. Use of ATP reagent

40 $\mu$L/well of 50% ATP reagent (CellTiter Glo 3D reagent, Promega) was dispensed into a 384-well plate. A pillar plate with cells according to Example 1-2 was combined with a well plate to which 50% ATP reagent was dispensed. After mixing well by shaking for 5 minutes, it was allowed to react at room temperature for 25 minutes. Luminescence was measured by using SPARK (TECAN). The degree of apoptosis (activity) was calculated in the drug-treated sample by converting the value that was not treated with the drug to 100 in the measured luminescence.

$$\text{Cell Viability (\%)} = (T/C) \times 100$$

T: Cell activity of drug-treated wells (ATP emission amount)

C: Cell activity of control well (ATP emission amount)

*1-3-2. Use of Calcein AM reagent*

40 μL/well of 4 μM Calcein AM reagent (MBD) was dispensed into a 384-well plate. After binding the pillar plate with cells to the well plate to which 4 μM Calcein AM reagent was dispensed, it was reacted in an incubator at 37°C for 1 hour. After transferring the pillar plate with cells stained with Calcein AM to a well plate into which PBS was dispensed, fluorescence was measured by using an ASFA Scanner (MBD). The degree of apoptosis (activity) was calculated in the drug-treated sample by converting the value of the drug-untreated sample to 100 in the measured fluorescence.

$$\text{Cell Viability } (\%) = (T/C) \times 100$$

T: Cell activity (fluorescence amount) of drug-treated well
C: Cell activity (fluorescence amount) of control well

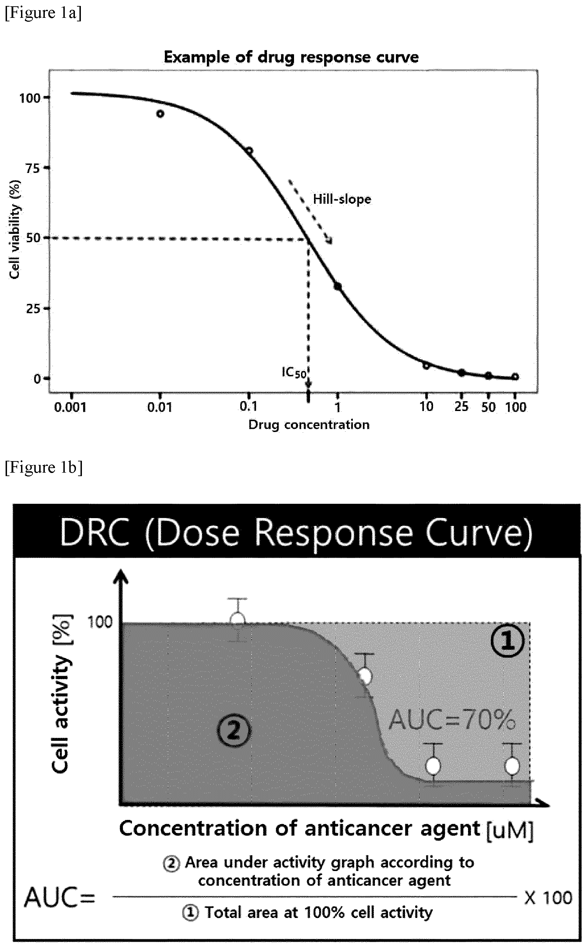

The degree of apoptosis of cancer cells according to the concentration of drug was calculated based on a drug response curve (DRC) and $IC_{50}$ which was calculated based on DRC, or an area under curve (AUC) (percentage of area when cell viability without drug treatment was maintained at 100%).
When the degree of apoptosis (activity) in the drug-treated sample was 50% or less, it was classified as a responder group that responded to the anticancer agent.
$IC_{50}$ means the concentration of drug required for the drug to inhibit 50% of cell activity under test conditions. In the DRC graph, when the function formula $F(x)$ of the Hill-slope section where the response curve of drug reactivity is a straight line is obtained and the value of X at which the value of $F(x)$ is 50 is obtained, this value corresponds to $IC_{50}$ (FIG. 1a).
The area under curve (AUC) represents the ratio of the area under the DRC curve ((2)) to the total area (①) when the cell activity is 100% (FIG. 1b).

1-4. Calculation of cell growth rate

*1-4-1. Use of ATP reagent*

40 μL/well of 50% ATP reagent (CellTiter Glo 3D reagent) was dispensed into a 384-well plate. The pillar plate containing control cells that were not treated with an anticancer agent according to Example 1-2 was combined with a well plate dispensed with 50% ATP reagent. After mixing well by shaking for 5 minutes, it was allowed to react at room temperature for 25 minutes. Luminescence was measured by using SPARK (TECAN). The cell growth rate was calculated by comparing the measured values of the drug treatment start date (3 days) and drug treatment end date (5 or 10 days).

$$\text{Cell growth rate } (\%) = (B/A) \times 100$$

A: Cell activity without drug treatment at the drug treatment start date
B: Cell activity without drug treatment at the drug treatment end date

*1-4-2. Use of Calcein AM reagent*

40 μL/well of 4 μM Calcein AM reagent (MBD) was dispensed into a 384-well plate. After binding the pillar plate with cells to the well plate to which 4 μM Calcein AM reagent was dispensed, it was reacted in an incubator at 37°C for 1 hour. After transferring the pillar plate with cells stained with Calcein AM to a well plate into which PBS was dispensed, fluorescence images were analyzed by using an ASFA Scanner (MBD).
The cell growth rate was calculated by comparing the fluorescence images at the drug treatment start date (3 days) and the drug treatment end date (5 or 10 days).

$$\text{Cell growth rate } (\%) = (B/A) \times 100$$

A: Cell activity without drug treatment at the drug treatment start date

B: Cell activity without drug treatment at the drug treatment end date

1-5. Derivation of anticancer agent susceptibility index and prediction of susceptibility to anticancer agent

It was attempted to improve the accuracy of prediction of susceptibility to an anticancer agent by considering the cell growth rate that differs for each cancer patient. Therefore, the Z-score of the drug response factor was calculated based on the degree of apoptosis calculated in Example 1-3, and the Z-score of the cell growth factor was calculated based on the cell growth rate calculated in Example 1-4.

Z-score Calculation Formula

$$Z = \frac{X - \mu}{\sigma}$$

Z: Z-score (standard score)
X: Raw score
$\mu$: Population mean
$\sigma$: Population standard deviation

For the population, at least 50 test data were collected based on anticancer agent susceptibility data of the same cancer type and the same drug as the sample to be tested.

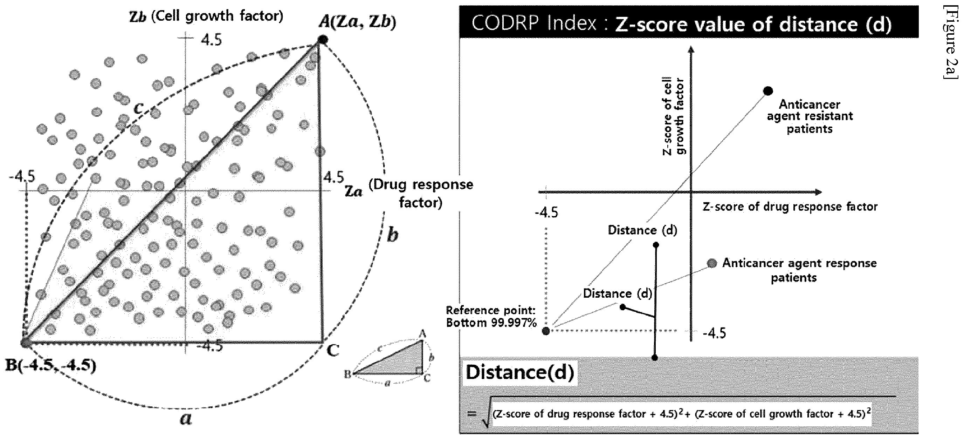

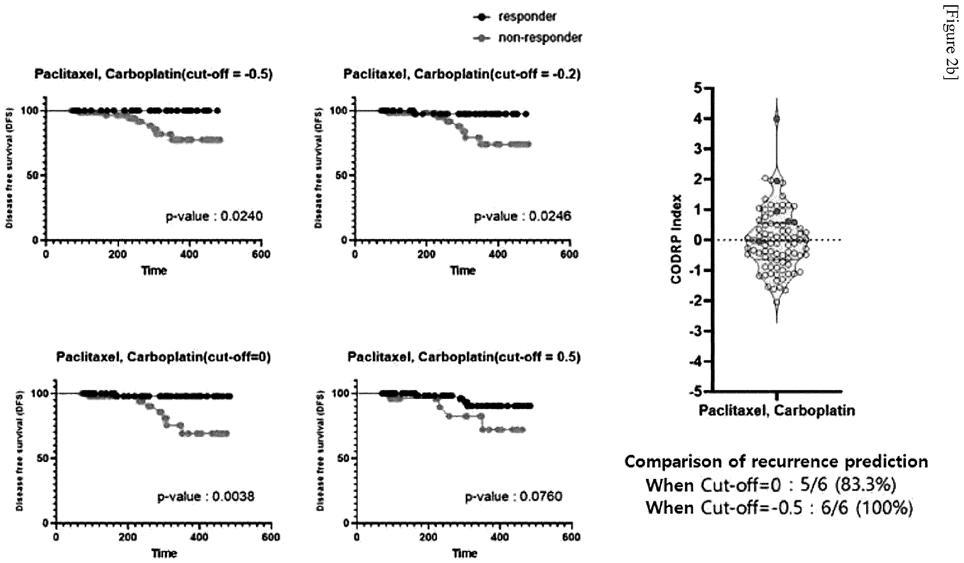

Specifically, the susceptibility to an anticancer agent can be predicted with reference to FIG. 2a, and in FIG. 2b, the X-axis represents the Z-score ($Z_a$) value of a drug response factor, and the Y-axis represents the Z-score ($Z_b$) value of a cell growth factor. $Z_a$ and $Z_b$ values are calculated from the sample, and the distance (d) to the calculated $Z_a$ and $Z_b$ values can be calculated by using (-4.5, -4.5) as a reference point to cover 99.997% of the population, and the Z-score of this distance was named the CODRP Index (Cancer Organoid-based Diagnosis Reactivity Prediction index, personalized cancer treatment responsiveness predictive index based on cancer organoid cell culture), which is an anticancer agent susceptibility index according to the present invention.

$$\textbf{CODRP index} = Z - score\ of\ \sqrt{(Z_a + 4.5)^2 + (Z_b + 4.5)^2}$$

In order to evaluate the effect of the drug, the predictive value of recurrence and the p-value of the disease free survival (DFS) of the responder group (sensitive group) and the non-responder group (resistant group) were analyzed.

As a result, as shown in FIG. 2b, when the cut-off value was adjusted to -0.5, -0.2, 0 and 0.5 on the DFS graph, 0 was evaluated as the lowest and the most significant, but when the predictive value of recurrence was compared, if the cut-off was 0, it was 83.3%, whereas if it was -0.5, it was 100%, and the p-value was also confirmed to be less than 0.05, which is routinely evaluated as significant. Therefore, as a result of considering both of these two factors, when the cut-off was -0.5, it was analyzed that the susceptibility to an anticancer agent could be most accurately predicted.

Therefore, when the CODRP Index was -0.5 or less, it was classified as the anticancer agent responder group (sensitive group), and when it was 0 or more, it was classified as the anticancer agent non-responder group (resistant group) to determine the susceptibility to an anticancer agent.

**Example 2. Verification of method for predicting susceptibility to anticancer agent**

The CODRP Index was calculated according to the method described in Example 1 from cancer cell samples that were isolated from a total of 21 cancer patients, including 9 types of breast cancer, 4 types of lung cancer and 8 types of colorectal cancer. The patients' clinical response evaluation was divided into a sensitive group and a resistant group according to the clinician's judgment results by applying the RECIST 1.1 criteria (clinical result-based data). The cancer cell samples and clinical response evaluation information were collected, IRB approval was obtained for research purposes to conduct the experiments, and written consent was obtained from patients, and the samples were used in the experiments.

In order to compare the excellence of the method for predicting susceptibility to an anticancer agent according to the present invention, the results of (1) Existing Method 1 ($IC_{50}$) (Comparative Example 1), (2) Existing Method 2 (AUC) (Comparative Example 2), (3) MBD Method 1 (CODRP index based on drug response factor ($IC_{50}$) and cell growth factor (cell growth rate) (Example 1) and (4) MBD Method 2 (CODPR index based on drug response factor (AUC) and cell growth factor (cell growth rate)) (Example 2) were analyzed, and the accuracy of the method for predicting susceptibility to an anticancer agent was analyzed by comparing the same with clinical response evaluation.

In the experiments of (1) Existing Method 1 ($IC_{50}$), (2) Existing Method 2 (AUC), (3) MBD Method 1 ($IC_{50}$ + growth) and (4) MBD Method 2 (AUC + Growth), the process of pre-treatment, three-dimensional culturing and the treatment of an anticancer agent was performed with the same process as in Example 1-1 and Example 1-2. In this case, (1) Existing Method 1 ($IC_{50}$) and (2) Existing Method 2 (AUC) were respectively converted into Z-scores, and then, these were classified into sensitive and non-sensitive groups based on -0.5.

The anticancer agents used for each type of cancer are as follows:

Breast cancer: Doxorubicin (selleckchem)
Lung cancer: Osimertinib, Afatinib (AdooQ Bioscience)
Colorectal cancer: 5-FU (Selleckchem) + Oxaliplatin (Sigma Aldrich)

2-1. Sensitivity

Sensitivity is the true positive rate, which was calculated as the ratio of clinically showing positive results in the experiment for the responder group.

2-2. Specificity

Specificity is the true negative rate, which was calculated the ratio of clinically showing negative results in the experiment for the non-responder group.

2-3. Analysis of results

As a result, as shown in FIGS. 3a to 3d, compared to the existing methods for predicting susceptibility to an anticancer agent by considering only $IC_{50}$ or AUC (Comparative Example 1, Comparative Example 2), in the methods for predicting susceptibility to an anticancer agent by using the CODPR index (Example 1, Example 2), it was confirmed that sensitivity and specificity were improved, which enabled more accurate prediction of the susceptibility to an anticancer drug.

In particular, among the methods for predicting susceptibility to an anticancer agent using the CODRP index according to the present invention, MBD Method 2 using AUC as a drug response factor corresponds to a positive (sensitive group) compared to MBD Method 1 using $IC_{50}$ as a drug response factor, but it was confirmed that the false negative error that was wrongly judged could be corrected, and the accuracy was higher in discriminating the true positive rate.

Example 3. Evaluation of excellence of CODRP index

The CODRP Index was calculated from cancer cell samples that were isolated from ovarian cancer patients according to the method described in Example 1. The patients' clinical response evaluation was divided into a sensitive group and a resistant group according to the judgment of the clinician (clinical result standard data). The cancer cell samples and clinical response evaluation information were collected, IRB approval was obtained for research purposes to conduct the experiments, and written consent was obtained from patients, and the samples were used in the experiments.

The experiments were conducted by using the ATP reagent in the same manner as in Example 1, and the anticancer agent efficacy was confirmed by converting the amount of light emitted by the control group into 100.

The accuracies of the prediction of susceptibility to an anticancer agent of the comparative examples and examples were compared and analyzed by using 110 samples isolated from ovarian cancer patients.

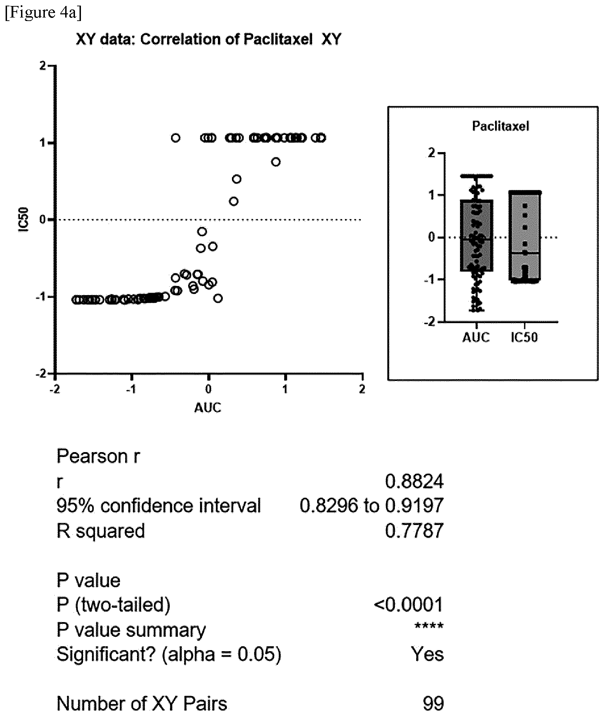

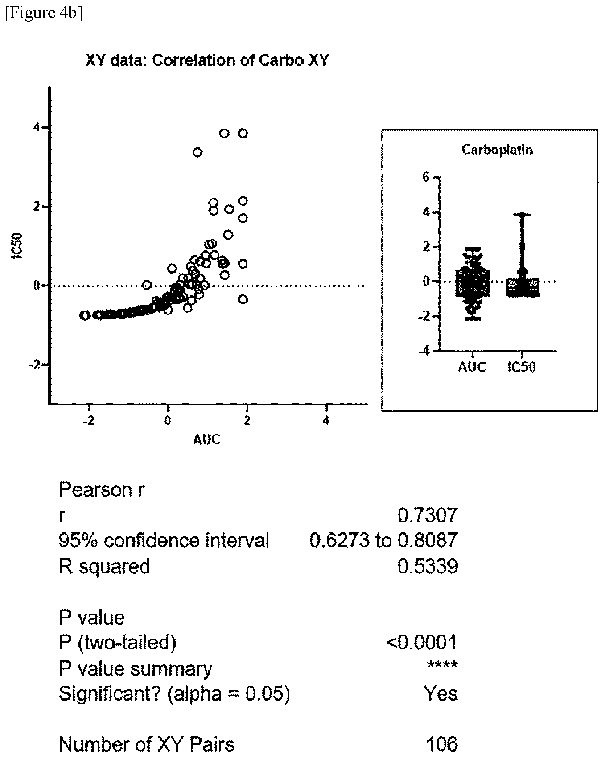

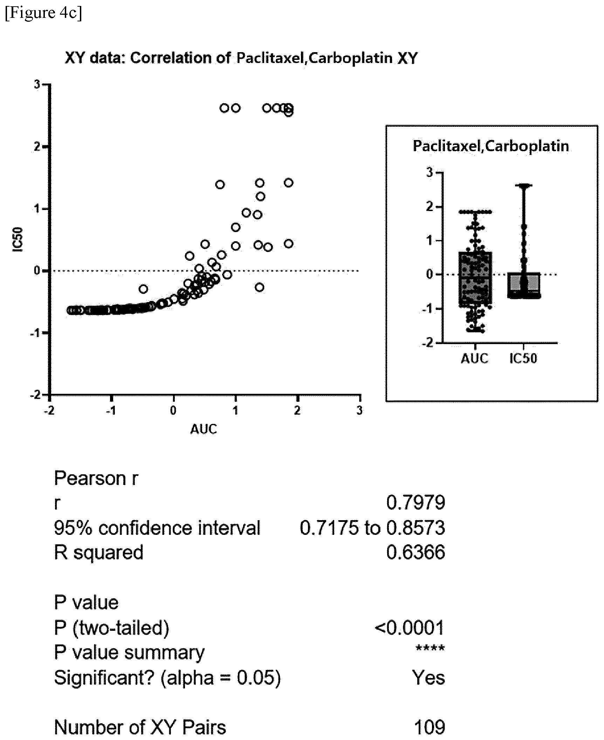

As a result of the analysis, as shown in FIGS. 4a to 4c, it was confirmed that the correlation between $IC_{50}$ and AUC was high (r = 0.7 or more, p-value <0.001) in the evaluation of the efficacy of anticancer agents Paclitaxel, Carboplatin and Paclitaxel + Carboplatin using ovarian cancer samples, and it was found that the area under curve (AUC) reflected drug responsiveness better than $IC_{50}$.

Meanwhile, the accuracies of the prediction of susceptibility to an anticancer agent according to the methods of Comparative Example 2 and Example 2 were compared with the ovarian cancer recurrence rate in patients who received the Carboplatin monotherapy or the Paclitaxel+Carboplatin combination therapy, which are the standard treatment method for ovarian cancer patients.

The Z-scores of Comparative Example 2 and Example 2 were compared, and specifically, for the AUC, data in which the average of the experimental values of the drug response curve was calculated as 0 and converted to a standard distribution value, and data in which the CODRP index was converted to a standard distribution value by calculating the average of AUC+ cell growth rate as 0 were shown as violin plots.

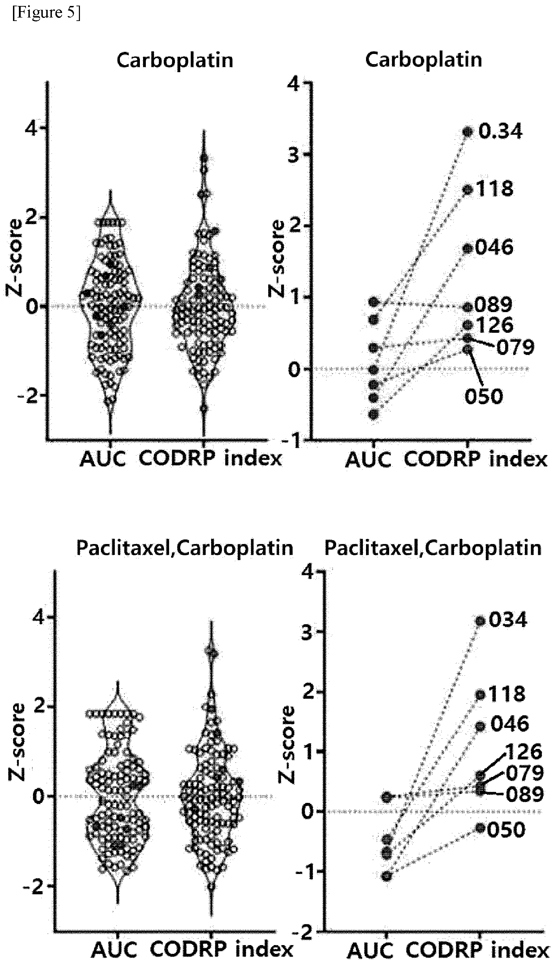

As a result, as shown in FIG. 5, when the susceptibility to an anticancer agent was predicted only by AUC, the distribution of relapsed patients was confirmed to exist even in the lower part (cases that were predicted to be sensitive to drugs) based on Z-score of 0, whereas when the susceptibility to an anticancer agent was predicted with the CODRP index, it was confirmed to be distributed on the upper part (cases that were predicted to have drug resistance) based on Z-score of 0, and thus, the CODRP index showed a higher predictive rate for recurrence patients (Carboplatin, AUC : 3/7, CODRP : 7/7; Pacli+Carbo, AUC : 2/7 ,CODRP : 6/7).

In addition, the disease free survivals (DFS) were compared by dividing into the responder group and the resistant group based on Z-score -0.5 in patients who received Carboplatin (AdooQ Bioscience) or the Paclitaxel (AdooQ Bioscience) + Carboplatin (Selleckchem) combination therapy, which is the standard treatment method for ovarian cancer patients. DFS is a Kaplan-Meier curve showing the period from the patient's specimen generation date (surgery date) to the recurrence date, that is, the presence of the event, that is, recurrence (Y-axis), according to the time elapsed (X-axis), and the graph shows a stepwise decreasing trend.

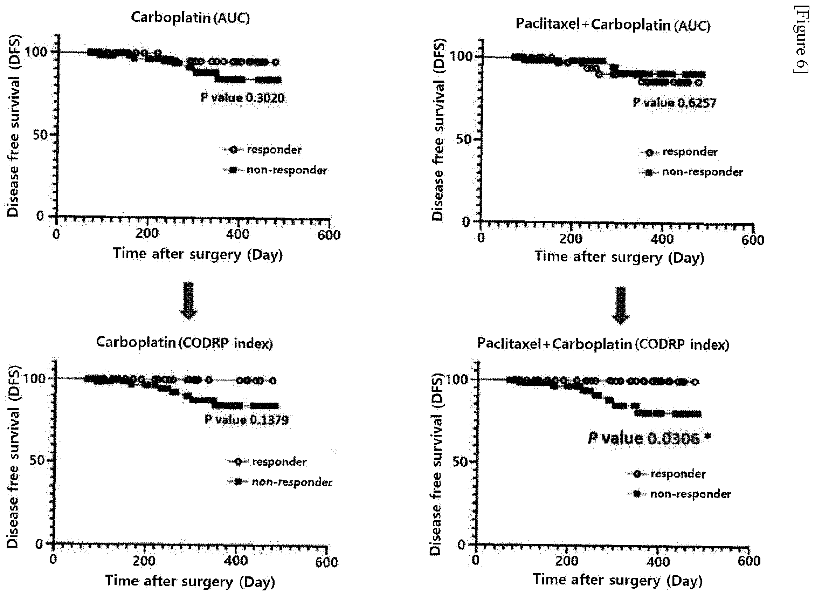

As a result of DFS analysis of Comparative Example 2 and Example 2, as shown in FIG. 6, the DFS p-values in the responder group and the resistant group for Carboplatin were shown at AUC 0.302 and CODRP index 0.1379, and the DFS p-value of the responder group and the resistant group for Paclitaxel+Carboplatin were shown at AUC 0.6257 and CODRP index 0.0306, and it was confirmed that when Example 2 was used instead of Comparative Example 2, the predictive rate of prognosis for anticancer agents was more excellent.

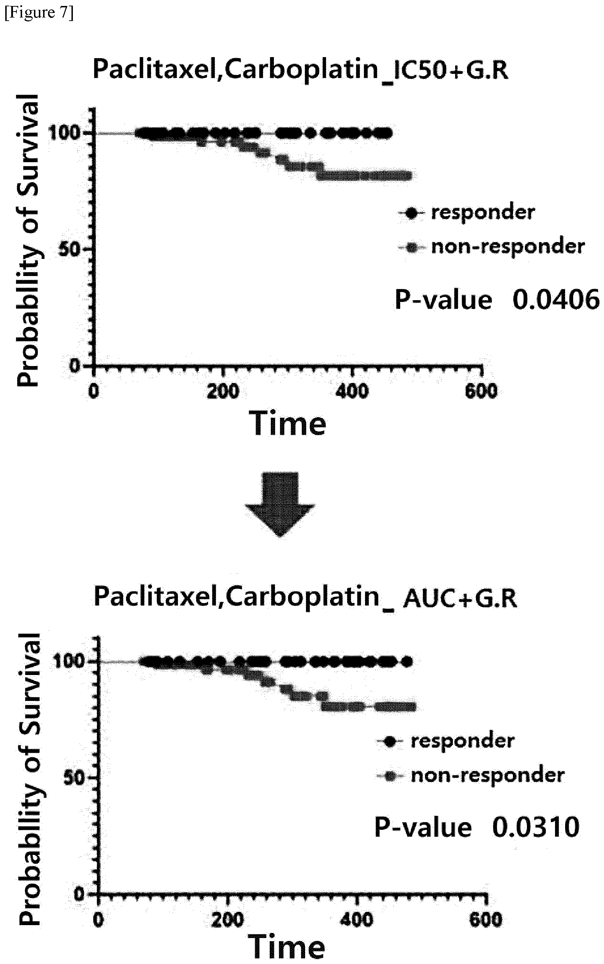

Additionally, in 110 samples obtained from ovarian cancer patients who received the Paclitaxel+Carboplatin combination therapy, DFS was compared by dividing into the responder group and the resistant group based on Z-score of -0.5. As a result, as shown in FIG. 7, particularly, Example 2 showed lower p-values of the responder group and the resistant group compared to Example 1, and it was confirmed that the accuracy of Example 2 was higher than that of Example 1.

Having described specific parts of the contents of the present invention in detail above, it is clear to those skilled in the art that these specific descriptions are only preferred exemplary embodiments, and the scope of the present invention is not limited thereby. Therefore, the substantial scope of the present invention will be defined by the appended claims and their equivalents.

## Claims

1. A method for providing information for predicting susceptibility to an anticancer agent, comprising the following steps:

   (a) culturing a biological sample isolated from a subject;
   (b) calculating the Z-score ($Z_a$) of a drug response factor after treating the cultured biological sample with an anticancer agent, and calculating the Z-score ($Z_b$) of a cell growth factor by culturing the biological sample without treating an anticancer agent; and
   (c) determining whether the anticancer agent response is positive or negative by integrating the calculated Z-score ($Z_a$) of the drug response factor and the Z-score ($Z_b$) of the cell growth factor.

2. The method of claim 1, wherein the biological sample is a cell sample isolated from a subject or a cell sample derived from a tissue isolated from a subject.

3. The method of claim 2, wherein the cell sample is three-dimensionally cultured in step (a).

4. The method of claim 1, wherein step (c) calculates the following CODRP index by integrating the calculated Z-score ($Z_a$) of the drug response factor and the Z-score ($Z_b$) of the cell growth factor:

$$\textbf{CODRP index} = Z - score\ of\ \sqrt{(Z_a + 4.5)^2 + (Z_b + 4.5)^2}$$

5. The method of claim 4, wherein the drug response factor is $IC_{50}$ or an area of a drug response curve (AUC).

6. The method of claim 4, wherein when the CODRP Index is -0.5 or less, the anticancer agent response is determined as positive, and when the CODRP Index is 0 or more, the anticancer agent response is determined negative, and when the CODRP Index is greater than -0.5 and less than 0, it is determined as deferred.

7. A system for predicting susceptibility to an anticancer agent, comprising:

   a receiving unit which is configured to receive cell experiment-based biological test data for a biological sample isolated from a subject;
   a processor which is connected to the receiving unit; and
   an output unit for outputting a result processed by the processor,
   wherein the processor calculates and integrates the Z-score ($Z_a$) of a drug response factor and the Z-score ($Z_b$) of a cell growth factor among the biological test data to provide prediction results of susceptibility to an anticancer agent for the subject.

8. The system of claim 7, wherein the processor calculates and integrates the Z-score ($Z_a$) of a drug response factor and the Z-score ($Z_b$) of a cell growth factor to calculate the following CODRP index:

$$\textbf{CODRP index} = Z - score\ of\ \sqrt{(Z_a + 4.5)^2 + (Z_b + 4.5)^2}$$

9. The system of claim 7, wherein the drug response factor is $IC_{50}$ or an area of a drug response curve (AUC)

10. The system of claim 8, wherein when the CODRP Index is -0.5 or less, the processor determines the anticancer agent response as positive, and when the CODRP Index is 0 or more, the processor determines the anticancer agent response as negative, and when the CODRP Index is greater than -0.5 and less than 0, the processor determines as deferred.

11. The system of claim 7, wherein the cell experiment-based biological test data comprises test data of at least one of the name of an anticancer agent, the concentration of an anticancer agent, a cell growth rate and the degree of apoptosis.

12. A computer-readable recording medium which records a program for implementing the system for predicting susceptibility to an anticancer agent according to any one of claims 7 to 11.

[Figure 1a]

[Figure 1b]

Zb (Cell growth factor)

A(Za, Zb)

4.5

-4.5

4.5 Za (Drug response factor)

c

b

-4.5

B(-4.5, -4.5)

C

a

CODRP Index : Z-score value of distance (d)

Z-score of cell growth factor

Anticancer agent resistant patients

Z-score of drug response factor

-4.5

Distance (d)

Anticancer agent response patients

Distance (d)

Reference point: Bottom 99.997%

-4.5

Distance(d)

$$= \sqrt{(\text{Z-score of drug response factor} + 4.5)^2 + (\text{Z-score of cell growth factor} + 4.5)^2}$$

[Figure 2b]

## MBD algorithm 1 (IC50+Cell growth rate)

| Measurement of susceptibility | | Clinical | |
|---|---|---|---|
| | | Sensitive group | Resistant group |
| | Sensitive group | 5 | 0 |
| | Resistant group | 1 | 3 |

Sensitivity=5/6*100=83%

Specificity=3/3*100=100%

## Existing algorithm (IC50)

| Measurement of susceptibility | | Clinical | |
|---|---|---|---|
| | | Sensitive group | Resistant group |
| | Sensitive group | 4 | 1 |
| | Resistant group | 2 | 2 |

Sensitivity=4/6*100=66%

Specificity=2/3*100=66%

## MBD algorithm 2 (AUC+Cell growth rate)

| Measurement of susceptibility | | Clinical | |
|---|---|---|---|
| | | Sensitive group | Resistant group |
| | Sensitive group | 6 | 0 |
| | Resistant group | 0 | 3 |

Sensitivity=6/6*100=100%

Specificity=3/3*100=100%

## Existing algorithm (AUC)

| Measurement of susceptibility | | Clinical | |
|---|---|---|---|
| | | Sensitive group | Resistant group |
| | Sensitive group | 5 | 1 |
| | Resistant group | 1 | 2 |

Sensitivity=5/6*100=83%

Specificity=2/3*100=66%

[Figure 3a]

EP 4 455 665 A1

**[Figure 3b]**

**EP 4 455 665 A1**

### MBD algorithm 1 (IC50+Cell growth rate)

| | | Clinical | |
|---|---|---|---|
| | | Sensitive group | Resistant group |
| Measurement of susceptibility | Sensitive group | 2 | 0 |
| | Resistant group | 0 | 2 |

Sensitivity=2/2*100=100%

Specificity=2/2*100=100%

### Existing algorithm (IC50)

| | | Clinical | |
|---|---|---|---|
| | | Sensitive group | Resistant group |
| Measurement of susceptibility | Sensitive group | 2 | 0 |
| | Resistant group | 1 | 1 |

Sensitivity=2/3*100=67%

Specificity=1/1*100=100%

### MBD algorithm 2 (AUC+Cell growth rate)

| | | Clinical | |
|---|---|---|---|
| | | Sensitive group | Resistant group |
| Measurement of susceptibility | Sensitive group | 2 | 0 |
| | Resistant group | 0 | 2 |

Sensitivity=2/2*100=100%

Specificity=2/2*100=100%

### Existing algorithm (AUC)

| | | Clinical | |
|---|---|---|---|
| | | Sensitive group | Resistant group |
| Measurement of susceptibility | Sensitive group | 2 | 0 |
| | Resistant group | 1 | 1 |

Sensitivity=2/3*100=67%

Specificity=1/1*100=100%

[Figure 3c]

Susceptibility results of anticancer agent for colorectal cancer          n(Number of patients) = 8

**MBD algorithm 1 (IC50+Cell growth rate)**

| | | Clinical | |
|---|---|---|---|
| | | Sensitive group | Resistant group |
| Measurement of susceptibility | Sensitive group | 3 | 1 |
| | Resistant group | 1 | 3 |

Sensitivity=3/4*100=75%
Specificity=3/4*100=75%

**Existing algorithm (IC50)**          n(Number of patients) = 8

| | | Clinical | |
|---|---|---|---|
| | | Sensitive group | Resistant group |
| Measurement of susceptibility | Sensitive group | 2 | 1 |
| | Resistant group | 2 | 3 |

Sensitivity=2/4*100=50%
Specificity=3/4*100=75%

**MBD algorithm 2 (AUC+Cell growth rate)**

| | | Clinical | |
|---|---|---|---|
| | | Sensitive group | Resistant group |
| Measurement of susceptibility | Sensitive group | 3 | 1 |
| | Resistant group | 1 | 3 |

Sensitivity=3/4*100=75%
Specificity=3/4*100=75%

**Existing algorithm (AUC)**

| | | Clinical | |
|---|---|---|---|
| | | Sensitive group | Resistant group |
| Measurement of susceptibility | Sensitive group | 2 | 1 |
| | Resistant group | 2 | 3 |

Sensitivity=2/4*100=50%
Specificity=3/4*100=75%

23

[Figure 3d]

EP 4 455 665 A1

n(Number of patients)=21

### MBD algorithm 1 (IC50+Cell growth rate)

| | | Clinical | |
|---|---|---|---|
| | | Sensitive group | Resistant group |
| Measurement of susceptibility | Sensitive group | 10 | 1 |
| | Resistant group | 2 | 8 |

Sensitivity=10/12*100=83%

Specificity=8/9*100=88%

n(Number of patients)=21

### Existing algorithm (IC50)

| | | Clinical | |
|---|---|---|---|
| | | Sensitive group | Resistant group |
| Measurement of susceptibility | Sensitive group | 8 | 2 |
| | Resistant group | 5 | 6 |

Sensitivity=8/13*100=61%

Specificity=6/8*100=75%

### MBD algorithm 2 (AUC+Cell growth rate)

| | | Clinical | |
|---|---|---|---|
| | | Sensitive group | Resistant group |
| Measurement of susceptibility | Sensitive group | 11 | 1 |
| | Resistant group | 1 | 8 |

Sensitivity=11/12*100=91%

Specificity=8/9*100=88%

### Existing algorithm (AUC)

| | | Clinical | |
|---|---|---|---|
| | | Sensitive group | Resistant group |
| Measurement of susceptibility | Sensitive group | 9 | 2 |
| | Resistant group | 4 | 6 |

Sensitivity=9/13*100=69%

Specificity=6/8*100=75%

[Figure 4a]

**XY data: Correlation of Paclitaxel XY**

Pearson r

| | |
|---|---|
| r | 0.8824 |
| 95% confidence interval | 0.8296 to 0.9197 |
| R squared | 0.7787 |

P value

| | |
|---|---|
| P (two-tailed) | <0.0001 |
| P value summary | **** |
| Significant? (alpha = 0.05) | Yes |

| | |
|---|---|
| Number of XY Pairs | 99 |

[Figure 4b]

**XY data: Correlation of Carbo XY**

Pearson r

| | |
|---|---|
| r | 0.7307 |
| 95% confidence interval | 0.6273 to 0.8087 |
| R squared | 0.5339 |

P value

| | |
|---|---|
| P (two-tailed) | <0.0001 |
| P value summary | **** |
| Significant? (alpha = 0.05) | Yes |

| | |
|---|---|
| Number of XY Pairs | 106 |

[Figure 4c]

XY data: Correlation of Paclitaxel,Carboplatin XY

Pearson r

| | |
|---|---|
| r | 0.7979 |
| 95% confidence interval | 0.7175 to 0.8573 |
| R squared | 0.6366 |

P value

| | |
|---|---|
| P (two-tailed) | <0.0001 |
| P value summary | **** |
| Significant? (alpha = 0.05) | Yes |

| | |
|---|---|
| Number of XY Pairs | 109 |

[Figure 5]

Carboplatin

Carboplatin

Paclitaxel,Carboplatin

Paclitaxel,Carboplatin

[Figure 6]

[Figure 7]

Paclitaxel,Carboplatin_IC50+G.R

Paclitaxel,Carboplatin_ AUC+G.R

## INTERNATIONAL SEARCH REPORT

| International application No. |
| --- |
| **PCT/KR2023/007615** |

### A. CLASSIFICATION OF SUBJECT MATTER

**G01N 33/50**(2006.01)i; **G16C 20/30**(2019.01)i; **G16C 20/80**(2019.01)i; **G16C 20/10**(2019.01)i; **G16B 5/00**(2019.01)i; **G16H 10/20**(2018.01)i

According to International Patent Classification (IPC) or to both national classification and IPC

### B. FIELDS SEARCHED

Minimum documentation searched (classification system followed by classification symbols)

G01N 33/50(2006.01); C12Q 1/48(2006.01); G01N 21/64(2006.01); G01N 21/65(2006.01); G01N 33/68(2006.01); G16C 20/10(2019.01); G16C 20/30(2019.01); G16H 50/70(2018.01); G16H 70/40(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 항암제(anti-cancer drug), 감수성(sensitivity), Z-score, CODRP index

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2343594 B1 (MBD CO., LTD.) 27 December 2021 (2021-12-27)<br>See paragraphs [0065]-[0067] and [0089]; and claims 1 and 10-11. | 1-2,7,9,11-12 |
| Y | | 3 |
| A | | 4-6,8,10 |
| Y | KR 10-2021-0108865 A (KOREA UNIVERSITY RESEARCH AND BUSINESS FOUNDATION et al.)<br>03 September 2021 (2021-09-03)<br>See abstract; paragraphs [0020]-[0037]; and claim 1. | 3 |
| A | CN 112435754 A (TIANJIN UNIVERSITY) 02 March 2021 (2021-03-02)<br>See entire document. | 1-12 |
| A | JP 5830249 B2 (SYSMEX CORPORATION) 09 December 2015 (2015-12-09)<br>See entire document. | 1-12 |

☑ Further documents are listed in the continuation of Box C.　　☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **05 September 2023** | **05 September 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/007615**

**C.  DOCUMENTS CONSIDERED TO BE RELEVANT**

| Category* | Citation of document, with indication, where appropriate, of the relevant passages | Relevant to claim No. |
|---|---|---|
| A | JP 2018-013453 A (INNOCO CORP) 25 January 2018 (2018-01-25)<br>See entire document. | 1-12 |

**INTERNATIONAL SEARCH REPORT**
Information on patent family members

International application No.

**PCT/KR2023/007615**

| Patent document cited in search report | | | Publication date (day/month/year) | Patent family member(s) | | | Publication date (day/month/year) |
|---|---|---|---|---|---|---|---|
| KR | 10-2343594 | B1 | 27 December 2021 | CN | 115116544 | A | 27 September 2022 |
| | | | | EP | 4064288 | A1 | 28 September 2022 |
| | | | | US | 2022-0310273 | A1 | 29 September 2022 |
| KR | 10-2021-0108865 | A | 03 September 2021 | KR | 10-2333275 | B1 | 03 December 2021 |
| CN | 112435754 | A | 02 March 2021 | CN | 112435754 | B | 08 April 2022 |
| JP | 5830249 | B2 | 09 December 2015 | EP | 2372362 | A2 | 05 October 2011 |
| | | | | EP | 2372362 | A3 | 12 October 2011 |
| | | | | EP | 2372362 | B1 | 27 March 2013 |
| | | | | JP | 2011-223986 | A | 10 November 2011 |
| | | | | US | 2011-0244497 | A1 | 06 October 2011 |
| JP | 2018-013453 | A | 25 January 2018 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 102343594

- KR 1020200055230